(19) Europäisches Patentamt
European Patent Office
Office européen des brevets

(11) Veröffentlichungsnummer: **0 143 747**
**B1**

(12) # EUROPÄISCHE PATENTSCHRIFT

(45) Veröffentlichungstag der Patentschrift: **02.05.90**

(51) Int. Cl.$^5$: **C 07 D 493/22**, A 01 N 43/22 // (C07D493/22, 313:00, 311:00, 311:00, 307:00)

(21) Anmeldenummer: **84810563.1**

(22) Anmeldetag: **19.11.84**

(54) **Neues Lacton-Derivat, Verfahren zu seiner Herstellung und seine Verwendung in der Schädlingsbekämpfung.**

(30) Priorität: **25.11.83 CH 6323/83**, **28.05.84 CH 2610/84**

(43) Veröffentlichungstag der Anmeldung: **05.06.85 Patentblatt 85/23**

(45) Bekanntmachung des Hinweises auf die Patenterteilung: **02.05.90 Patentblatt 90/18**

(84) Benannte Vertragsstaaten: **BE CH DE FR GB IT LI NL SE**

(56) Entgegenhaltungen:

**THE JOURNAL OF ANTIBIOTICS, Oktober 1980, Seiten 1120-1127; YO TAKIGUCHI et al.: "Milbemycins, a new family of macrolide antibiotics: fermentation, isolation and physico-chemical properties"**

(73) Patentinhaber: **CIBA-GEIGY AG**
**Klybeckstrasse 141**
**CH-4002 Basel (CH)**

(72) Erfinder: **Burckhardt, Urs, Dr.**
**Rittergasse 29**
**CH-4051 Basel (CH)**

**EP 0 143 747 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Milbemycin-Derivat der Formel I, seine Herstellung und seine Verwendung als Zwischenprodukt zur weiteren Derivatisierung von Milbemycin-Makroliden sowie seine Verwendung zur Bekämpfung von Schädlingen, ferner Schädlingsbekämpfungsmittel, die diese Verbindung als Wirkstoff enthalten.

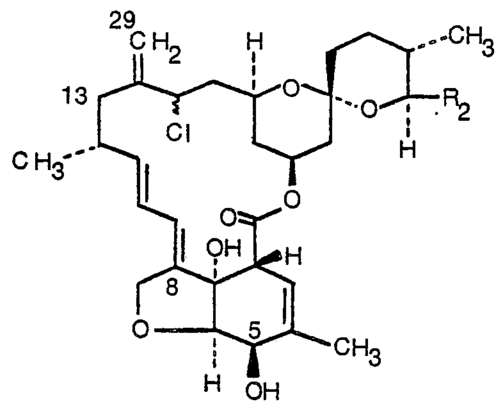

(I)

$R_2 = CH_3$, $C_2H_5$, iso$C_3H_7$ oder sek.$C_4H_9$.

In J. Antibiotics *33*, No. 10, Oct. 1980, Seiten 1120—1127, wird eine Reihe von Milbemycinen offenbart, denen insektizide und akarizide Eigenschaften zugeschrieben werden.

Die Verbindung I wird durch selektive Chlorierung eines Milbemycin-Derivats der Formel II im Temperaturbereich von −10°C bis +60°C, vorzugsweise 0°C bis 40°C, in Gegenwart eines Lösungsmittels hergestellt:

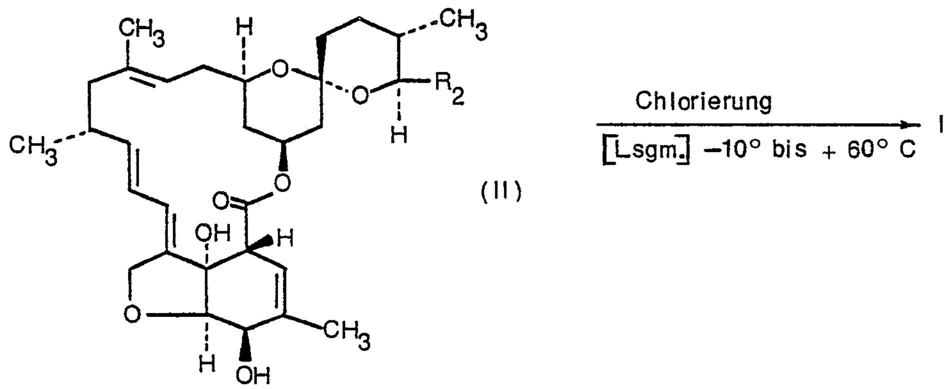


$R_2 = CH_3$ Milbemycin $A_3$ aus US—A—3,950,360.
$R_2 = C_2H_5$ Milbemycin $A_4$ aus US—A—3,950,360.
$R_2 =$ iso$C_3H_7$ Milbemycin D aus US—A—4,346,171.
$R_2 =$ sec.$C_4H_9$ 13-Desoxi-22,23-dihydro-C-076-$B_{1a}$-aglycon, oder 13-Desoxi-22,23-dihydro-avermectin-$B_{1a}$-aglykon aus US—A—4,173,571.

Die Reaktion stellt eine Chlorierung in 15-Stellung und die gleichzeitige Bildung einer exocylischen Doppelbindung in 29-14-Stellung dar. Für solche selektiven Chlorierungen an einer terminalen trisubstituierten Doppelbindung, wie sie die im Milbemycin vorliegende Methylalken-Gruppierung darstellt, hat sich als selektives Chlorierungsmittel, und zwar insbesondere bei gleichzeitiger Anwesenheit von Hydroxylgruppen im Molekül, unterchlorige Säure (=HOCl) erwiesen [Sh.G.Hedge et al. Tetrahedron Letters *21*, pp. 441—444 (1980)]. In der Regel setzt man HOCl als wässrige Lösung eines Alkali- oder Erdalkali-Hypochlorits ein und setzt HOCl mit einer schwachen Säure frei.

Es hat sich gezeigt, dass man für die erfindungsgemässe Chlorierung auch Sulfurylchlorid ($SO_2Cl_2$) verwenden kann.

Als Reaktionsmedium kommen inerte Lösungsmittel oder Lösungsmittelgemische in Frage, beispielsweise aliphatische oder aromatische, vorzugsweise halogenierte Kohlenwasserstoffe wie Benzol, Toluol, Chlorbenzol, Kohlenstofftetrachlorid, Chloroform, Dichlormethan; Aether oder ätherartige Verbindungen wie Diäthyläther, Dioxan, Tetrahydrofuran; Nitrile wie Acetonitril; und Wasser. Dichlormethan wird als Lòsungsmittel bevorzugt.

Die Reaktion kann in homogener oder heterogener Phase (z.B. Dichlormethan/Wasser) verlaufen und ergibt in beiden Fällen überraschend hohe Ausbeuten.

Die Verbindung der Formel I stellt ein vielseitiges Zwischenprodukt zur Gewinnung weiterer Milbemycin-Derivate dar, wobei die 5—OH—Gruppe vorteilhaft geschützt wird. Als Schutzgruppen kommen Acylreste (wie Acetyl, Propionyl, Benzoyl, Methansulfonyl, Tolylsufonyl u.a.) und vor allem Silylreste in Frage. Als letztere sind besonders Trimethylsilyl, Methyldiphenysilyl, tris(tert.Butyl)silyl, Diphenyl-tert.butylsilyl, bis(Isopropyl)methylsilyl und tert.Butyldimethylsilyl zu nennen. Die 5—OH—Gruppe kann auch als Benzylether oder Methoxiethoximethylether vorliegen.

Die Verbindung der Formel I eignet sich zur Bekämpfung von Schädlingen an Tieren und Pflanzen, darunter tierparasitären Ecto- und Endo-Parasiten. Zu letzteren zählen unter der Ordnung Acarina insebesondere Schädlinge der Familien Ixodidae, Dermanyssidae, Sarcoptidae, Psoroptidae; die Ordnungen Mallophaga; Siphonoptera, Anoptera (z.B. Familie der Haematopinidae); unter der Ordnung Diptera insbesondere Schädlinge der Familien Muscidae, Calliphoridae, Oestrididae, Tabanidae, Hippoboscidae, Gastrophilidae.

Die Verbindung I ist auch einsetzbar gegen Hygiene-Schädlinge insbesondere der Ordnungen Diptera mit den Familien Sarcophagiae, Anophilidae, Culicidae; der Ordnung Orthoptera (z.B. Familie Blattidae) und der Ordnung Hymenoptera (z.B. Familie Formicidae).

Die Verbindung I besitzt auch nachhaltige Wirksamkeit bei pflanzenparasitären Milben und Insekten. Bei Spinnmilben der Ordnung Acarina ist sie wirksam gegen Eier, Nymphen und Adulte von Tetranychidae (Tetranychus spp. und Panonychus spp.). Hohe Aktivität besitzt sie bei den saugenden Insekten der Ordnungen Homoptera, insbesondere gegen Schädlinge der Familien Aphididae, Delphacidae, Cicadellidae, Psyllidae, Loccidae, Diaspididae und Eriophyidae (z.B. die Rostmilbe auf Citrusfrüchten); der Ordnungen Hemiptera; Heteroptera und Thysanoptera; sowie bei den pflanzenfressenden Insekten der Ordnungen Lepidoptera; Coleoptera; Diptera und Orthoptera.

Sie ist ebenfalls als Bodeninsektizid gegen Schädlinge im Erdboden geeignet.

Die Verbindung der Formel I ist daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindung der Formel I ist auch wirksam gegen Pflanzen-Nematoden der Arten Meloidogyne, Heterodera, Pratylenchus, Ditylenchus, Radiopholus, Rhizoglyphus und andere.

Darüberhinaus ist die Verbindung besonders gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: *Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Unicinaria, Toxascaris* und *Parascaris*. Der besondere Vorteil der Verbindung der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum griefen den Intestinaltrakt des Wirtstiers an, während andere der Arten Haemonchus und Osteragia im Magen und solche der Art Dictyocaulus im Lungegewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Die Verbindung der Formel I ist gegen diese Parasiten wirksam.

Sie ist ferner zur Bekämpfung von humanpathogenen Parasiten geeignet, unter denen als typische, im Verdauungstrakt vorkommende Vertreter solche der Arten *Ancylostoma, Necator, Ascaris, Strongyloides, Trichinella, Capillaria, Trichuris* und *Enterobius* zu nennen sind. Wirksam ist die Verbindung vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Exo-Intestinalkanal infizieren.

Die Verbindung der Formel I wird in unveränderter Form oder vorzugsweise zusammen mit der in der Formulierungstechnik üblichen Hilfsmitteln eingesetzt und wird daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Gissen werden gleich wie die Art der Mittel den angestrebten Zielen und den gegebenen Verhältnissen entsprechend gewählt.

Die Verbindung der Formel I wird bei Warmblütern in Aufwandmengen von 0,01 bis 50 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestelt, z.B. durch inniges Vermischen und/oder Vermahlen der Wirkstofe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder

-äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sajaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorrillonit oder Attapulgit. Zur Verbesserung der physikalischen Eingenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder an ionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höherern Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegenbenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die suflonierten Benzimidazolderivate enthalten vorzugsweise 2-Sulfonsäuregruppen und einen Fettsäurerest mit 8—22 C-Atomen, Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Aethylenoxid-Adduktes oder Phospholipide in Frage.

Die in der Formulierungstechnik gebräuchlichen Tenside sind u.a. in folgender Publikation beschrieben:

"McCutcheon's Detergents and Emulsifiers Annual" MC Publishing Corp., Ringwood, New Jersey, 1982.

Die pestiziden Zubereitungen enthalten in der Regel 0,01 bis 95%, insbesondere 0,1 bis 80%, Wirkstoff der Formel I, 5 biss 99,99% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel mit 1—10,000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiel 1 für das $\Delta^{29,14}$—15H—15—Chlor-Milbemycin D

Bei Raumtemperatur werden 2,23 g (= 4 mMol) Milbemycin-D in 100 ml analysenreinem Dichlormethan gelöst und mit einer Lösung von 820 mg (= 8 mMol) $Ca(OCl)_2$ (ca. 70%ig) in 10 ml dest. Wasser versetzt. In die entstandene Suspension werden unter energischem Rühren von Zeit zu Zeit kleine Stücke von festem $CO_2$ zur Freisetzung der unterchlorigen Säure zugefügt, die zusammengenommen etwa das Dreifache der stöchiometrisch benötigten Menge ausmachen. Nach 2,5 bis 3 Std. wird die Reaktion beendet. Man trennt die Phasen, trocknet die organische Phase mit $Na_2SO_4$, filtriert, engt ein und erhält 2,3 g der Verbindung I (85% d.Th.) Smp. 137 bis 140°C (Zers.).

Herstellungsbeispiel 2 für das gleiche Präparat

4,45 g (= 8 mMol) Milbemycin-D werden in 100 ml analysenreinem Dichlormethan gelöst und unter schwachem Rühren bei Raumtemperatur mit 0,65 ml (= 16 mMol) Sulfurylchlorid versetzt. Die klare farblose Lösung wird 15 Std. bei ca. 25°C gerührt, dann dreimal mit gesättigter wässriger $NaHCO_3$-Lösung und einmal mit Wasser gewaschen und nach Abtrennung der wässrigen Lösungüber $Na_2SO_4$ getrocknet, filtriert und eingeengt. Man erhält 4,8 g der Verbindung I als amorphen gelben Festkörper, der durch Flash-Chromatographie gereinigt wird: 2 g Verb. I (= 42% der Theorie), Smp. 139 bis 140°C (Zers.).

Herstellungsbeispiel 3 für das $\Delta^{29,14}$—15H—15—Chlor-Milbemycin-$A_3$

Bei Raumtemperatur werden 1,06 g (= 2 mMol) Milbemycin-$A_3$ in 50 ml analysenreinem Dichlormethan gelöst und mit einer Lösung von 410 mg (= 4 mMol) $Ca(OCl)_2$ (ca. 70%ig) in 5 ml dest. Wasser versetzt. In die entstandene Suspension werden unter energischem Rühren von Zeit zu Zeit kleine Stücke von festem $CO_2$ zur Freisetzung der unterchlorigen Säure zugefügt, die zusammengenommen etwa das Dreifache der stöchiometrisch benötigten Menge ausmachen. Nach 2,5 bis 3 Std. wird die Reaktion beendet. Man trennt die Phasen, trocknet die organische Phase mit $Na_2SO_4$, filtriert, engt ein und erhält einen Rückstand, der durch Flash-Chromatographie (Laufmittel $CH_2Cl_2$/Methanol 1000:3) gereinigt wird. Man erhält 250 mg (22% d.Th.) des gewünschten $A_3$ -Derivats, Mol.-Gew. 563,13. Massenspektrum m/e: *562* ($M^+$), 434, 312, 181, 151.

Herstellungsbeispiel 4 für das $\Delta^{29,14}$—15H—15—Chlor-Milbemycin-$A_4$

Bei Raumtemperatur werden 1,08 g (= 2 mMol) Milbemycin-$A_4$ in 50 ml analysenreinem Dichlormethan gelöst und mit einer Lösung von 410 mg (= 4 mMol) $Ca(OCl)_2$ (ca. 70%ig) in 5 ml dest. Wasser versetzt. In die entstandene Suspension werden unter energischem Rühren von Zeit zu Zeit kleine Stücke von festem $CO_2$ zur Freisetzung der unterchlorigen Säure zugefügt, die zusammengenommen etwa das Dreifache der stöchiometrisch benötigten Menge ausmachen. Nach 2,5 bis 3 Std. wird die Reaktion beendet. Man trennt die Phasen, trocknet die organische Phase mit $Na_2SO_4$, filtriert, engt ein zur Trockne und reinigt den verbliebenen Rückstand durch Flash-Chromatographie (Laufmittel Dichlormethan). Man erhält 460 mg (398.8% d.Th.) des gewünschten $A_4$ -Derivats. Mol.-Gew. 577,16. Massenspektrum m/e: *576* ($M^+$) 448, 312, 195, 167, 151.

Herstellungsbeispiel 5 für das 13-Desoxi-$\Delta^{29,14}$—15H—15—chlor-22,23-dihydro-avermectin-$B_{1a}$-aglykon

Nach Art des Beispiels 1 oder 2 lassen sich aus 228 mg (= 0,4 mMol) 13-Desoxi-22,23-dihydro-avermectin-$B_{1a}$-aglykon 119 mg 13-Desoxi-$\Delta^{29,14}$—15H—15—chlor-22,23-dihydro-avermectin-$B_{1a}$-aglykon gewinnen.

Formulierungsbeispiele für den Wirkstoff der Formel I (% = Gewichtsprozent)

| *Spritzpulver* | a) | b) | c) |
|---|---|---|---|
| Wirkstoff | 25% | 50% | 75% |
| Na-Ligninsulfonat | 5% | 5% | — |
| Na-Laurylsulfat | 3% | — | 5% |
| Na-Diisobutylnapthalinsulfonat | — | 6% | 10% |
| Octylphenolpolyäthylenglykoläther (7—8 Mol Aeo) | — | 2% | — |
| Hochdisperse Kiselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| *Emulsions-Konzentrat* | |
|---|---|
| Wirkstoff | 10% |
| Octylphenolpolyäthylenglykoläther (4—5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| *Stäubemittel* | a) | b) |
|---|---|---|
| Wirkstoff | 5% | 8% |
| Talkum | 95% | — |
| Kaolin | — | 92% |

Man erhält anwendungsfertige Stäubemittel, indem der Wirkstoff mit dem Träger vermischt und auf einer geeigneten Mühle vermahlen wird.

| *Extruder Granulat* | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschlissend im Luftstrom getrocknet.

Falls die Verbindung der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wierderholt verabreicht werden, wobei die einzelnen Gaben je nach Tierart vorzugsweise zwischen 0.1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mttel können auch dem Futter oder den Tränken zugesetzt werden. Das Fertigfutter enthält die Wirkstoffkombinationen vorzugsweise in einer Konzentration von 0,005 bis 0,1 Gew.-%. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, kann die Verbindung der Formel I bzw. sie enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

B-1. Insektizide Frassgit-Wirkung bei Spodoptera littoralis

Eingetopfte Baumwollpflanzen im 5-Blatt-Stadium werden mit einer acetonisch/wässrigen Versuchslösung besprüht, die 3; 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24° C und 60% relativer Luftfeuchtigkeit durchgeführt. Auswertungen und Zwischenauswertungen auf moribunde Tiere, Wachstum und Larven und Frassschäden erfolgen nach 24 Std., 48 Std. und 72 Stunden.

Die Verbindungen der Formel I erzielten bereits bei einer Wirkstoffkonzentration von 3 ppm eine vollständige Abtötung nach 24 Stunden.

B-2. Wirkung gegen pflanzenschädigende Akariden OP-sesnsible Tetranychus urticae

Die Primärblätter von Bohnenpflanzen (Phaseolus vulgaris) werden 16 Std. vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25° C.

Nach sieben Tagen wird unter dem Binokular auf Prozentsatz mobiler Stadien (Adulte und Nymphen) und auf vorhandene Eier ausgewertet.

Die Verbindungen der Formel I erzielten bereits bei einer Wirkstoffkonzentration von 0,4 ppm vollständige Abtötung.

B-3. Wirkung gegen $L_1$-Larven von Lucilia sericata

1 ml einer wässrigen Suspension der zu prüfenden Aktivsubstanz werden so mitt 3 ml eines speziellen Larvenzuchtmediums bei ca. 50° C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen der Formel I erzielten mit 250 ppm eine Wirkung von 100%.

B-4. Akarizide Wirkung gegen Boophilus microplus (Biarra-Stamm)

Auf einer PVC-Platte wird waagerecht ein Klebestreifen so befestigt, dass darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µm einer Flüssigkeit injiiziert, die eine 1:1-Mischung von Polyethylenglykol und Aceton darstellt und in der eine bestimmte Wirkstoffmenge von wahlweise 1, 0.1 oder 0.01 µg pro Zecke gelöst ist. Kontrolltiere erhalten eine wirkstoffreie Injektion. Nach der Behanlung werden die Tiere unter Normalbedingungen in einem Insektarium bei ca. 28° C und 80% rel. Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90%) Eier ablegen, die nicht schlupffähig sind.

Die Verbindungen der Formel I erzielen eine $IR_{90}$ von 0.1 µg.

B-5. Versuch an mit Nematoden (Haemonchus concortus und Trichostronglyus colubriformis) infizierten Schafen.

Der Wirkstoff wird als Suspension formuliert mit einer Magensonde oder durch Injektion in den Pansen eines Schafes gegeben, das mit Haemonchus concortus und Trichostrongylus columbriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formel I bei 2 mg/kg behandelt wurden, zeigten im Vergleich zu unbehandelten, aber infizierten Vergleichsgruppen keinen Nematodenbefall (= komplette Reduktion der Wurmeier im Kot).

B-6. Kontakwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80% tote bzw. abgefallene Blattläuse ausgewertet. Nur bei dieser Wirkungshöhe wird ein Präparat als wirksam eingestuft.

Die Verbindungen der Formel I erzielten bei einer Konzentration von 12,5 ppm eine vollständige Abtötung (= 100%).

B-7. Larvizidwirkung gegen Aedes aegypti

Auf die Oberfläche von 150 ml Wasser, das sich in einem Behälter befindet, wird soviel einer 0,1%igen acetonischen Lösung des Wirkstoffes pipettiert, dass Konzentrationen von wahlweise 10 ppm; 3,3 ppm und 1,6 ppm erhalten werden. Nach Verdunsten des Acetons wird der Behälter mit ca. 30—40 3 Tage alten Aëdes-Larven beschickt. Nach 1, 2 und 5 Tagen wird die Mortalität geprüft.

Die Verbindungen der Formel I bewirkten in diesem Test bei einer Konzentration von 1,6 ppm bereits nach einem Tag eine vollständige Abtötung sämtlicher Larven.

**Patentansprüche**

1. Milbemycin-Derivat der Formel I

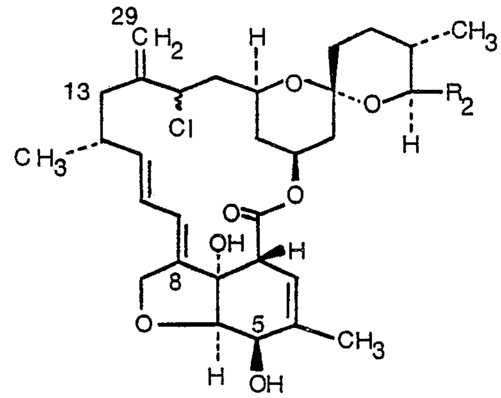

(I)

worin $R_2$ Methyl, Aethyl, Isopropyl oder sek.Butyl bedeutet.

2. Milbemycin-Derivat der Formel I, worin $R_2$ Methyl oder Aethyl bedeutet.

3. Milbemycin-Derivat der Formel I, worin $R_2$ Isopropyl bedeutet.

4. Verfahren zur Herstellung der Verbindung der Formel I gemäss Anspruch 1, gekennzeichnet durch selektive Chlorierung eines Milbemycin-Derivats der Formel II

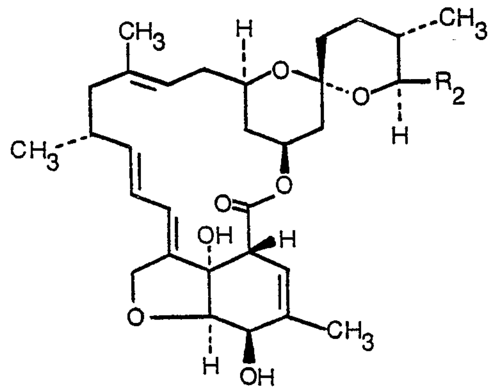

(II)

worin $R_2$ Methyl, Aethyl, Isopropyl oder sek.Butyl bedeutet, in einer inerten Lösungsmittelphase bei −10°C bis +60°C mit wahlweise unterchloriger Säure (HOCl) oder Sulfurylchlorid ($SO_2Cl_2$).

5. Verfahren gemäss Anspruch 4, gekennzeichnet durch einen Temperaturbereich von 0°C bis +40°C.

6. Verfahren gemäss Anspruch 4, gekennzeichnet durch Verwendung von Dichlormethan als Lösungsmittel.

7. Schädlingsbekämpfungsmittel enthaltend als mindestens einen Wirkstoff die Verbindung der Formel I gemäß Ansprüch 1 zusammen mit üblichen Trägerstoffen und/oder Verteilungsmitteln.

8. Mittel gemäss Anspruch 7 enthaltend die Verbindung der Formel I, worin $R_2$ Methyl oder Aethyl bedeutet.

9. Mittel gemäss Anspruch 7, enthaltend die Verbindung der Formel I, worin $R_2$ Isopropyl bedeutet.

10. Verwendung der Verbindung der Formel I des Anspruchs 1 zur Bekämpfung von pflanzenparasitären Insekten und Acarina.

11. Verwendung der Verbindung der Formel I des Anspruchs 1 zur Bekämpfung von pflanzenparasitären Nematoden.

12. Verbindung der Formel I des Anspruchs 1 zur Verwendung bei der Bekämpfung von Ekto- und Endoparasiten am tierischen Warmblüter.

13. Verfahren zur Bekämpfung von Schädlingen an Pflanzen, dadurch gekennzeichnet, dass man die Verbindung der Formel I gemäss Anspruch 1 auf oder in die Pflanzen appliziert.

14. Verbindung der Formel I gemäss Anspruch 1 zur Verwendung bei der Bekämpfung von Schädlingen an Tieren durch Applikation besagter Verbindung auf oder in die Tiere.

15. Verbindung der Formel I gemäss Anspruch 1 zur Verwendung bei der Bekämpfung endoparasitärer, bei Säugetieren und Geflügel auftretenden Nematoden.

16. Verwendung einer Verbindung der Formel I gemäss Anspruch 1 zur Herstellung eines Arzneimittels zur Bekämpfung von Ekto- und Endoparasiten am tierischen Warmblüter.

**Revendications**

1. Dérivé de Milbemycine répondant à la formule I

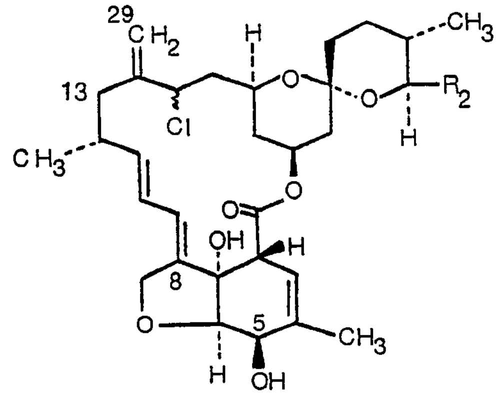

(I)

dans laquelle $R_2$ représente un groupe méthyle, éthyle, isopropyle ou sec-butyle.

2. Dérivé de la Milbemycine de formule I dans laquelle $R_2$ représente un groupe méthyle ou éthyle.

3. Dérivé de la Milbemycine de formule I dans laquelle $R_2$ représente un groupe isopropyle.

4. Procédé de préparation du composé de formule I selon la revendation 1, caractérisé en ce que l'on chlore sélectivement un dérivé de Milbemycine répondant à la formule II

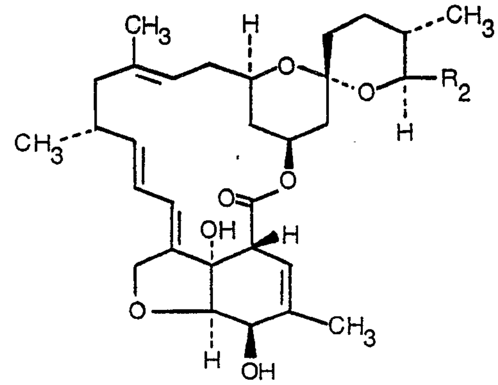

(II)

dans laquelle $R_2$ représente un groupe méthyle, éthyle, isopropyle ou sec-butyle, dans une phase solvant inerte entre −10 et +60°C, à volonté à l'aide de l'acide hypochloreux (HOCl) ou du chlorure de sulfuryle ($SO_2Cl_2$).

5. Procédé selon la revendication 4, caractérisé en ce que l'on opère dans un intervalle de température de 0 à +40°C.

6. Procédé selon la revendication 4, caractérisé en ce que le solvant utilisé est le dichlorométhane.

7. Produit pesticide contenant au moins une substance active consistant en un composé de formule I selon la revendication 1, avec des véhicules et/ou des diluants usuels.

8. Produit selon la revendication 7, contenant le composé de formule I dans laquelle $R_2$ représente un groupe méthyle ou éthyle.

9. Produit selon la revendication 7, contenant le composé de formule I dans laquelle $R_2$ représente un groupe isopropyle.

10. Utilisation du composé de formule I de la revendication 1 dans la lutte contre les insectes et acariens parasitant les végétaux.

11. Utilisation du composé de formule I de la revendication 1 pour la lutte contre les nématodes parasitant les végétaux.

12. Composé de formule I de la revendication 1 pour l'utilisation dans la lutte contre les ecto- et endo-parasites des animaux à sang chaud.

13. Procédé pour combattre les parasites sur les végétaux, caractérisé en ce que l'on applique le composé de formule I de la revendication 1 sur ou dans les végétaux.

14. Composé de formule I selon la revendication 1, pour l'utilisation dans la lutte contre les parasites des animaux par application dudit composé sur les animaux ou administration dudit composé aux animaux.

15. Composé de formule I selon la revendication 1, pour l'utilisation dans la lutte contre les nématodes endoparasitaries des mammifères et de la volaille.

16. Utilisation d'un composé de formule I selon la revendication 1 pour la préparation d'un médicament servant à combattre les ecto- et endo-parasites des animaux à sang chaud.

**Claims**

1. A milbemycin derivative of the formula I

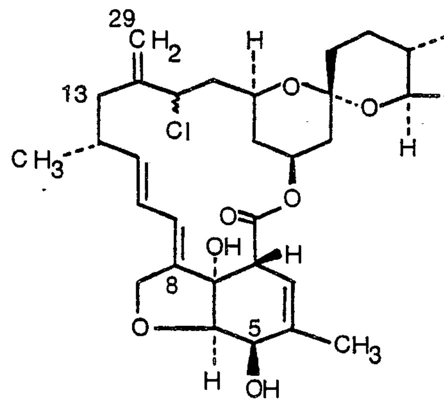

(I)

wherein $R_2$ is methyl, ethyl, isopropyl or sec.-butyl.

2. A milbemycin derivative of the formula I, wherein $R_2$ is methyl or ethyl.

3. A milbemycin derivative of the formula I, wherein $R_2$ is isopropyl.

4. A process for the preparation of a compound of the formula I according to claim 1, which comprises selectively chlorinating a milbemycin derivative of the formula II.

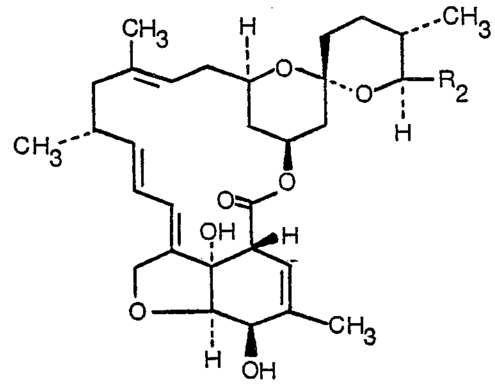

(II)

wherein $R_2$ is methyl, ethyl, isopropyl or sec.-butyl, either with hypochlorous acid (HOCl) or with sulfuryl chloride ($SO_2Cl_2$), in an inert solvent phase at from −10° to +60°C.

5. A process according to claim 4, wherein the temperature range is from 0° to +40°C.

6. A process according to claim 4, wherein dichloromethane is used as solvent.

7. A pesticidal composition containing, as at least one active ingredient, the compound of the foirmula I according to claim 1 together with customary carriers and/or distributing agents.

8. A composition according to claim 7, which contains the compound of the formula I, wherein $R_2$ is methyl or ethyl.

9. As composition according to claim 7, which contains the compound of the formula I, wherein $R_2$ is isopropyl.

10. The use of the compound of the formula I of claim 1 for controlling Acarina and insects which are plant parasites.

11. The use of the compound of the formula I of claim 1 for controlling nematodes which are plant parasites.

12. A compound of the formula I of claim 1 for use in controlling ecto- and endo-parasites in warm-blooded animals.

13. A method of controlling pests of plants, which comprises applying the compound of the formula I according to claim 1 to or into the plants.

14. A compound of the formula I according to claim 1 for use in controlling pests of animals by applying said compound to or into the animals.

15. A compound of the formula I according to claim 1 for use in controlling endoparasitic namatodes that occur in mammals and fowl.

16. The use of a compound of the formula I according to claim 1 for the preparation of a medicament for controlling ecto- and endo-parasites in warm-blooded animals.